# EUROPÄISCHE PATENTSCHRIFT

(11) **EP 1 880 682 B1**
(45) Veröffentlichungstag und Bekanntmachung des Hinweises auf die Patenterteilung: **28.04.2010**
(21) Anmeldenummer: 07117713.3
(22) Anmeldetag: 28.05.2004
(51) Int. Cl.: A61B 17/14, B23D 51/10

(54) **Chirurgisches Sägeblatt**
Surgical saw blade
Lame chirurgicale

(30) Priorität: 28.05.2003 DE 10325391
(43) Veröffentlichungstag der Anmeldung: 23.01.2008
(62) Teilanmeldung aus: 04739500.9
(73) Patentinhaber: Aesculap AG, 78532 Tuttlingen (DE)
(72) Erfinder: Blust, Edgar, 78126, Königsfeld (DE); Butsch, Werner, 78589, Dürbheim (DE); Gassner, Stefan, 78194, Immendingen-Hattingen (DE); Hagen, Thomas, 78532, Tuttlingen (DE); Häusler, Rainer, 78532, Tuttlingen (DE); Högerle, Alois, 78532, Tuttlingen (DE); Kleinwächter, Timo, 78532, Tuttlingen (DE)
(74) Vertreter: Hoeger, Stellrecht & Partner Patentanwälte

(56) Entgegenhaltungen:
- DE-A- 2 935 732
- US-A- 5 735 866
- US-A- 5 846 244
- US-A1- 2006 009 796
- US-A1- 2007 119 055

## Beschreibung

Die Erfindung betrifft ein chirurgisches Sägeblatt mit einem Haltekörper und einer Sägeeinrichtung, welche an dem Haltekörper gehalten ist und relativ zu dem Haltekörper beweglich ist, wobei die Sägeeinrichtung einen schwenkbaren, eine oszillierende Bewegung durchführbaren Sägekopf umfasst.

Solche chirurgischen Sägeblätter sind beispielsweise aus der DE 101 00 630 C1 und der DE 202 11 397 U1 bekannt.

Aus der DE 26 11 720 A1 ist ein Bandsägen-Skalpell bekannt. Die DE 27 58 380 A1 offenbart eine Kette für ein motorisch angetriebenes Trenngerät für den medizinischen Bereich.

Die WO 99/20184 A1 und die US 5,846,244 offenbaren chirurgische Sägen.

Aus der DE 29 35 732 A1 ist eine Vorrichtung zum Antrieb einer Kette eines Präzisions-Skalpells mit einem Kettenrad zum Antrieb und zur Umlenkung der Kette in ihrem einen Scheitelbereich und mit einem Klingenteil, das zumindest im Bereich des Kettenrads zwei Seitenwände aufweist, zwischen denen die Kette läuft und zwischen denen auch das Kettenrad läuft, sowie mit einer Lagerbohrung zur drehbaren Lagerung eines Bundes des Kettenrads bekannt. Mindestens eine der Seitenwände weist im Bereich des Kettenlaufs eine Austrittsöffnung für Schnittgut-Mulm auf.

Aus der US 5,735,866 (Basis für den Oberbegriff des Anspruchs 1) ist ein Sägeblatt bekannt, welches zwei Bereiche zueinander aufweist, die relativ zueinander einstellbar sind, um die Gesamtlänge des Sägeblatts variieren zu können.

Chirurgische Sägeblätter werden insbesondere im Zusammenhang mit Oszillationssägen für orthopädische Zwecke eingesetzt, beispielsweise um Platz zu schaffen für eine Knieendoprothese.

Der Erfindung liegt die Aufgabe zugrunde, ein chirurgisches Sägeblatt der eingangs genannten Art zu schaffen, welches für verbesserte Handhabungseigenschaften sorgt.

Diese Aufgabe wird bei dem eingangs genannten chirurgischen Sägeblatt erfindungsgemäß dadurch gelöst, dass die Sägeeinrichtung über den Haltekörper an einen Antrieb zur Bewegung der Sägeeinrichtung gekoppelt ist, wobei der Haltekörper bei einem Oszillationssägevorgang nicht bewegt ist, dass der Haltekörper einen Kopplungsbereich umfasst, über den ein externer Antrieb wirksam an ein Kraftübertragungselement oder an Kraftübertragungselemente zur Bewegung der Sägeeinrichtung koppelbar ist, dass das oder die Kraftübertragungselemente zwischen gegenüberliegenden äußeren Oberflächen des Haltekörpers angeordnet sind und dass der Haltekörper an einem Sägeende mit einem Schlitz versehen ist, in welchem der Sägekopf schwenkbar ist.

Dadurch, dass die Sägeeinrichtung relativ zu dem Haltekörper beweglich ist, um so eine Sägebewegung durchzuführen, ist die bewegte Masse geringer, als wenn der gesamte Haltekörper bewegt wird. Damit lässt sich die Belastung eines Getriebes einer Antriebssäge verringern und dadurch wiederum lässt sich die Lebensdauer des Sägengetriebes erhöhen. Chirurgische Sägen werden üblicherweise im Akkubetrieb eingesetzt. Durch die erfindungsgemäße Lösung wird die bewegte Masse reduziert, so dass sich durch den verringerten Energiebedarf auch die Sägenlaufzeit erhöhen lässt.

Die Sägeeinrichtung umfasst einen schwenkbaren Sägekopf. Ein solcher schwenkbarer Sägekopf lässt sich in eine Oszillationsbewegung versetzen, um so einen Sägeschnitt einbringen zu können. Durch einen bezüglich einem Haltekörper schwenkbaren Sägekopf ist es möglich, den Haltekörper einzuspannen oder zu führen, da der Haltekörper selber für eine Sägebewegung nicht beweglich sein muss. Es lässt sich dann auch ein relatives langes Sägeblatt ausbilden, da nur der Sägekopf bewegt werden muss und nicht das ganze Sägeblatt. Da nur der Sägekopf bewegt werden muss, ist dadurch auch der Weg festgelegt, so dass auch bei langen Sägeblättern kein größerer Oszillationsweg vorgesehen werden muss. Durch das Vorsehen eines beweglichen und insbesondere oszillierbaren Sägekopfs besteht auch die Möglichkeit, den Sägekopf entsprechend des durchzuführenden Sägevorgangs anzuordnen. Beispielsweise kann dieser vorne an einem Haltekörper angeordnet sein oder seitlich.

Die Sägeeinrichtung ist angetrieben, um so einen Sägeschnitt einbringen zu können.

Ein Antrieb und insbesondere ein Antriebsmotor zur Bewegung der Sägeeinrichtung ist dabei vorzugsweise extern angeordnet, das heißt in einer Säge angeordnet, an welche das Sägeblatt gekoppelt ist.

Die Sägeeinrichtung ist über den Haltekörper an einen Antrieb zur Bewegung der Sägeeinrichtung gekoppelt. Dadurch lässt sich die Handhabung vereinfachen, wenn insbesondere vorgesehen ist, dass durch die Fixierung des Haltekörpers an der Säge gleichzeitig eine Ankopplung des Antriebs erfolgt. Es lässt sich so auch erreichen, dass Kraftübertragungselemente (Zwischengetriebe), die benötigt werden, um den Antrieb der Säge an die Sägeeinrichtung zu koppeln, verdeckt in dem Haltekörper unterbringbar sind, so dass insbesondere die Verunreinigungsanfälligkeit verringert ist und auch ein Bediener geschützt ist.

Der Haltekörper umfasst einen Kopplungsbereich, über den ein externer Antrieb wirksam an Kraftübertragungselemente zur Bewegung der Sägeeinrichtung koppelbar ist.

Kraftübertragungselemente sind zwischen gegenüberliegenden äußeren Oberflächen des Haltekörpers angeordnet, so dass sie gegenüber dem Außenraum verdeckt angeordnet sind.

Vorzugsweise sind an dem Haltekörper Kraftübertragungselemente zur Bewegung der Sägeeinrichtung angeordnet. Dadurch lässt sich das Drehmoment des Antriebs der Säge in einer räumlichen Distanz zu der Sägeeinrichtung in den Haltekörper einkoppeln.

Besonders günstig ist es, wenn die Kraftübertragungselemente im Haltekörper angeordnet sind. Dadurch sind sie vom Außenraum geschützt, das heißt besser gegen Verunreinigungen geschützt.

In einer einfachen Ausführungsform umfasst der Kopplungsbereich eine Öffnung in dem Haltekörper. Durch diese Öffnung hindurch kann dann ein Kopplungselement der Säge an Kraftübertragungselemente des Sägeblattes angreifen.

Zur Einbringung eines Sägeschnittes ist es günstig, wenn der Haltekörper an einem Sägeende eine größere Breite aufweist als an einem gegenüberliegenden Ende. Es lässt sich dadurch ein langer Sägeschnitt einbringen, wobei andererseits die Oberfläche des Haltekörpers, mit welcher diese in einen Sägespalt eintaucht, minimiert ist, da von dem Sägeende weg die Breite des Haltekörpers abnimmt. Dadurch wiederum lässt sich sicherstellen, dass Sägespäne aus dem Sägespalt abführbar sind.

Es ist vorgesehen, dass sich die Sägeeinrichtung an einem Sägeende des Haltekörpers quer zu einer Längsrichtung des Haltekörpers bewegt. Eine solche Bewegungsform lässt sich auf einfache Weise erreichen, wobei sich lange Sägeschnitte einbringen lassen.

Es ist vorgesehen, dass die Sägeeinrichtung eine oszillierende Bewegung durchführt.

Insbesondere ist der Sägekopf schwenkbar in dem Haltekörper gehalten. Dadurch lässt sich der Mechanismus für die Schwenkbewegung bzw. Oszillationsbewegung des Sägekopfs in dem Haltekörper selber geschützt anordnen.

Aus dem gleichen Grund ist es günstig, wenn der Sägekopf von dem Haltekörper umgriffen ist.

Der Sägekopf weist dabei eine Zahnreihe auf, die dann oszillierend beweglich ist, um den Schnittspalt zu erzeugen.

Es ist günstig, wenn die Zähne der Zahnreihe mindestens so hoch sind wie der Haltekörper. Dadurch lässt sich ein sicherer Schnitt einbringen. Darüber hinaus ist der Späneanteil, der in den Haltekörper eindringen kann, minimiert.

Für eine gute Schnittwirkung ist es günstig, wenn Spitzen der Zähne der Zahnreihe auf einer Kreislinie liegen.

Insbesondere liegt dann der Mittelpunkt der Kreislinie auf einer Schwenkachse für den Sägekopf.

Ganz besonders vorteilhaft ist es, wenn eine Antriebsachse für den Schwenkantrieb des Sägekopfs und eine Schwenkachse beabstandet sind. Die Antriebsachse ist üblicherweise eine Rotationsachse, um die ein Antriebselement rotiert, welches beispielsweise durch einen Motor angetrieben ist. Die Schwenkachse ist die Schwenkachse eines Schwenklagers, über welches der Sägekopf an dem Haltekörper gehalten ist. Durch eine Beabstandung der Antriebsachse und der Schwenkachse ergeben sich eine Vielzahl von Möglichkeiten zur Dimensionierung des entsprechenden Sägeblatts und zur Anordnung des Sägekopfs an dem Haltekörper. Beispielsweise kann der Sägekopf vorne oder seitlich am Haltekörper angeordnet sein.

Es ist dann ein Umsetzungstrieb zur Umsetzung eines Antriebsrotationsbewegung in eine Sägekopf-Schwenkbewegung (Oszillationsbewegung) vorgesehen. Über diesen Umsetzungsantrieb wird die Distanz wischen der Antriebsachse und der Schwenkachse überbrückt. Ferner wird eine Rotationsbewegung in eine Oszillationsbewegung umgesetzt.

Es ist konstruktiv günstig, wenn der Umsetzungstrieb als Exzentertrieb ausgebildet ist. Es lässt sich dann auf einfache Weise eine Rotationsbewegung in eine Oszillationsbewegung umsetzen.

Günstigerweise ist der Mechanismus des Umsetzungstriebs im Haltekörper angeordnet. Dadurch sind bewegliche Teile in dem Haltekörper geschützt und der Haltekörper lässt sich über seine Oberflächen führen oder einspannen.

Ein Sägekopf lässt sich auf konstruktiv einfache Weise über einen Pleueltrieb als Umsetzungstrieb antreiben, welcher an dem Haltekörper angeordnet ist. Dieser Pleueltrieb stellt dann ein Zwischengetriebe zwischen dem Antrieb der Säge und dem Sägekopf dar.

Üblicherweise ist der Sägekopf so an dem Haltekörper angeordnet, dass eine Schwenkbewegung relativ zu einem vorderen Ende des Haltekörpers durchführbar ist. Insbesondere ist dann eine Schwenkbewegung bzw. Oszillationsbewegung um eine Achse durchführbar, welche einen Durchstoßpunkt der Antriebsachse und der Schwenkachse verbindet.

Es ist aber auch möglich, dass der Sägekopf so an dem Haltekörper angeordnet ist, dass eine Schwenkbewegung relativ zu einem seitlichen Ende des Haltekörpers durchführbar ist. Eine Achse, um welche eine Oszillationsbewegung erfolgt, fällt dann nicht mehr mit einer Verbindungsachse zwischen Durchstoßpunkten der Antriebsachse und der Schwenkachse zusammen, sondern liegt quer zu dieser.

Bei einer weiteren Ausführungsform ist ein erster Sägekopf und ein zweiter Sägekopf vorgesehen, wobei die beiden Sägeköpfe relativ zueinander beweglich sind. Es lässt sich dann gleichzeitig sowohl mit dem ersten Sägekopf als auch mit dem zweiten Sägekopf eine Schnittbewegung durchführen. Dadurch lässt sich die effektive Frequenz bei der Erzeugung eines Schnittspaltes erhöhen.

Besonders vorteilhaft ist es, wenn der erste Sägekopf den zweiten Sägekopf zumindest teilweise umgibt. Dadurch lässt sich dann der zweite Sägekopf relativ zu dem Haltekörper beweglich führen, um so eine Schnittbewegung durchzuführen. Wenn der erste Sägekopf fest an dem Haltekörper sitzt und den zweiten Sägekopf umgibt, dann ist über einen Innenraum des Haltekörpers Platz bereitgestellt, um ein Zwischengetriebe zum Antrieb des zweiten Sägekopfes aufnehmen zu können.

Insbesondere weist der erste Sägekopf eine Ausnehmung auf, in welcher der zweite Sägekopf angeordnet ist.

Vorzugsweise ist der zweite Sägekopf relativ zum Haltekörper beweglich.

Es ist dann vorteilhaft, wenn erster und zweiter Sägekopf gegenläufig beweglich sind. Dadurch lässt sich ein Schnittspalt mit erhöhter Sägefrequenz herstellen.

Die nachfolgende Beschreibung bevorzugter Ausführungsformen dient im Zusammenhang mit der Zeichnung der näheren Erläuterung der Erfindung. Es zeigen:
- Figur 1: eine Draufsicht auf ein erstes Ausführungsbeispiel eines chirurgischen Sägeblattes, welches nicht unter die Ansprüche fällt;
- Figur 2: eine Schnittansicht des chirurgischen Sägeblattes gemäß Figur 1 längs der Linie 2-2;
- Figur 3: eine Draufsicht auf ein zweites Ausführungsbeispiel eines chirurgischen Sägeblattes, welches nicht unter die Ansprüche fällt;
- Figur 4: eine Vorderansicht auf ein Sägeende des chirurgischen Sägeblattes gemäß Figur 3;
- Figur 5: eine Schnittansicht eines Ausführungsbeispiels eines erfindungsgemäßen chirurgischen Sägeblattes;
- Figur 6: eine Schnittansicht längs der Linie 6-6 gemäß Figur 5;
- Figur 7: eine Schnittansicht eines weiteren Ausführungsbeispiels eines erfindungsgemäßen chirurgischen Sägeblattes;
- Figur 8: eine Vorderansicht auf ein Sägeende des chirurgischen Sägeblattes gemäß Figur 7;
- Figur 9: eine Schnittansicht eines Ausführungsbeispiels eines erfindungsgemäßen chirurgischen Sägeblattes, welches nicht unter die Ansprüche fällt;
- Figur 10: ein Ausführungsbeispiel eines Umsetzungstriebs, welcher bei dem chirurgischen Sägeblatt gemäß den Figuren 5 und 6 einsetzbar ist;
- Figur 11: ein weiteres Beispiel für einen Umsetzungstrieb;
- Figur 12: ein weiteres Beispiel für einen Umsetzungstrieb;
- Figur 13: ein weiteres Beispiel für einen Umsetzungstrieb;
- Figur 14: eine Seitenansicht des Umsetzungstriebs gemäß Figur 14 und
- Figur 15: ein weiteres Ausführungsbeispiel eines Umsetzungstriebs.

Chirurgische Sägeblätter werden insbesondere für orthopädische Zwecke im Zusammenhang mit akkubetriebenen Sägen und insbesondere Oszillationssägen eingesetzt. Beispielsweise muss beim Einsatz von Knieendoprothesen zuvor Platz für die Prothese geschaffen werden.

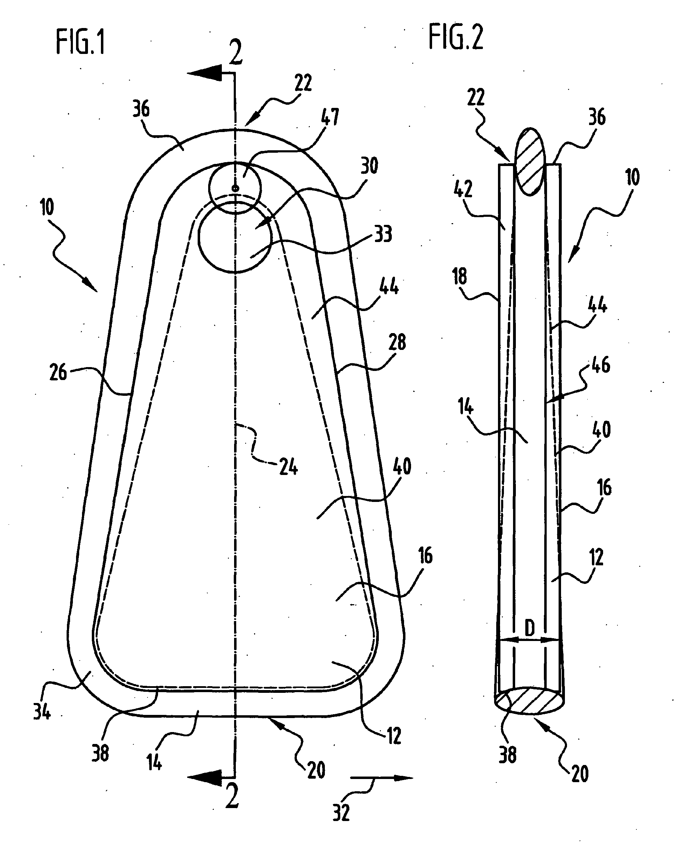

Ein erstes Ausführungsbeispiel eines chirurgischen Sägeblattes zum Einsatz mit einer Säge, welches nicht unter die Ansprüche fällt und in den Figuren 1 und 2 gezeigt und dort als Ganzes mit 10 bezeichnet ist, umfasst einen Haltekörper 12, an welchem eine Sägeeinrichtung 14 gehalten ist. Über den Haltekörper 12 ist das Sägeblatt 10 an der Säge fixierbar, wobei die Sägebewegung des Sägeblattes 10 durch einen Antrieb der Säge angetrieben ist.

Der Haltekörper 12 hat gegenüberliegende im Wesentlichen parallele Oberflächen 16, 18 zwischen welchen der Haltekörper 12 gebildet ist. Die Oberflächen 16, 18 sind im Wesentlichen eben, wobei eine Oberflächenstruktur vorgesehen sein kann, welche konvex gekrümmte Ausnehmungen aufweist. Eine solche Oberflächenstruktur ist in der DE 101 00 630 C1 beschrieben, auf die ausdrücklich Bezug genommen wird.

Der Haltekörper 12 weist ein Sägeende 20 und ein gegenüberliegendes Ende 22 auf. An dem Sägeende 20 ist der Haltekörper 12 breiter als an dem gegenüberliegenden Ende 22, so dass bezogen auf eine Längsachse 24 des Haltekörpers 12 gegenüberliegende Seiten 26, 28 des Haltekörpers 12 in einem spitzen Winkel von dem Ende 22 zu dem Sägeende 20 orientiert sind.

Der Haltekörper 12 weist einen Kopplungsbereich 30 (Anschluss) für die Säge auf, über welchen eben das Sägeblatt 10 an der Säge fixierbar ist. Der Kopplungsbereich umfasst vorzugsweise eine Öffnung 33.

Die Sägeeinrichtung 14 ist relativ zu dem Haltekörper 12 beweglich, wobei eine Bewegungsrichtung 32 der Sägeeinrichtung 14 im Bereich des Sägeendes 20 quer zur Oberflächennormale der Oberflächen 16, 18 ist und quer zur Längsachse 24 und insbesondere rechtwinklig zu dieser ist.

Die Sägeeinrichtung 14 ist bei dem Sägeblatt 10 als Sägeband 34 (Sägedraht) ausgebildet. Dieses Sägeband 34 ist umlaufend um den Haltekörper 12 geführt. Dazu sind die Seiten 26, 28 und die Stirnseite 36 des Haltekörpers 12 zumindest teilweise als Führungen ausgebildet. Das Ende 22 liegt dabei an der Stirnseite 36 und das Sägeende 20 an einer der Stirnseite 36 gegenüberliegenden Stirnseite 38.

Es kann beispielsweise vorgesehen sein, dass randseitig zwischen Deckelelementen 40 und 42, an welchen jeweils die Oberflächen 16 und 18 gebildet sind, eine zwischen den Seiten 26, 28 und 36 umlaufende Nut 44 angeordnet ist, in welcher das Sägeband 34 geführt ist.

Das Sägeband 34 ist eine Endloseinrichtung, das heißt ein Endlosband, welches an einem durch die Nut 44 gebildeten Seitenbereich des Haltekörpers 12 geführt ist.

Vorzugsweise ist es vorgesehen, dass das Sägeband 34 in einem Querschnitt in senkrecht aufeinander stehenden Richtungen unterschiedliche Durchmesserlängen aufweist, das heißt in einem solchen Querschnitt unterschiedliche Breiten aufweist. Beispielsweise weist das Sägeband 34 einen elliptischen Querschnitt auf. An der großen Halbachse ist dann die Durchmesserlänge größer als an der kleinen Halbachse. Das Sägeband 34 hat dabei solche Abmessungen, dass die Durchmesserlänge an der großen Halbachse größer ist als eine Dicke D des Haltekörpers 12 zwischen den Oberflächen 16 und 18, und dass die Durchmesserlänge an der kleinen Halbachse kleiner ist als die Dicke D und insbesondere höchstens der (maximalen) Ausdehnung der Nut 44 entspricht.

An dem Sägeende 20 ist das Sägeband 34 so aufgestellt, dass seine Höhenabmessung in der Dickenrichtung des Haltekörpers 12 größer ist als die Dicke D. Dadurch lässt sich ein Schnittspalt erzeugen, der breiter ist als die Dicke D.

An dem Haltekörper 12 und insbesondere den Seiten 26, 28 ist eine Umlenkeinrichtung 46 angeordnet, um eben das Sägeband 34 so umzulenken, dass der größere Durchmesser des Sägebandes 34 am Sägeende 20 parallel zu diesem Sägeende 20 ausgerichtet ist. Bezogen auf die Bewegungsrichtung 32 ist dabei beispielsweise an der Seite 26 die Umlenkeinrichtung 46 so ausgebildet, dass sie das Sägeband 34 für das Sägeende 20 aufrichtet, während die Umlenkeinrichtung 46 an der gegenüberliegenden Seite 28 so ausgebildet ist, dass sie das Sägeband 24 wieder umlegt, um dieses in der Nut 44 führen zu können.

Das Sägeband 34 läuft in der Nut 44 um den Seitenumfang des Haltekörpers 12 um. Diese Bewegung des Sägebandes 34 ist durch die Säge angetrieben. Ein Antrieb der Säge kann dabei direkt auf das Sägeband 34 wirken oder es können an dem Haltekörper 12 Kraftübertragungselemente wie Reibräder 47 vorgesehen sein, auf welche der Antrieb wirkt und über die die Bewegung des Sägebandes 34 in der Bewegungsrichtung 32 antreibbar ist. Der Sägenantrieb kann über die Öffnung 33 auf ein Reibrad 47 wirken und dies antreiben, wobei das Reibrad wiederum auf das Sägeband 34 wirkt und dessen Bewegung antreibt. Die Kraftübertragungselemente des Haltekörpers 12 sind dann insbesondere verdeckt zwischen den Deckelementen 40, 42 angeordnet und über die Öffnung 33 kann der Antrieb der Säge an diese Kraftübertragungselemente gekoppelt werden (in den Figuren 1 und 2 nicht gezeigt).

Zur Einbringung eines Sägeschnitts wird das Sägeband 34 als Sägeeinrichtung 14 relativ zu dem Haltekörper 12 in einer umlaufenden Bewegung um einen umlaufenden Seitenrand des Haltekörpers 12 bewegt. Da nur das Sägeband 34 bewegt wird und nicht der Haltekörper 12, entspricht die bewegte Masse der Masse des Sägebandes 34. Diese Masse ist im Vergleich zu der des Haltekörpers 12 gering. Dadurch lässt sich die Belastung des Getriebes der Säge verringern und die Laufzeit der Säge lässt sich erhöhen (üblicherweise werden chirurgische Sägen über Akkus betrieben).

Statt einer umlaufenden Bewegung des Sägebandes 34 kann auch eine oszillierende Bewegung vorgesehen sein, das heißt das Sägeband 34 bewegt sich am Sägeende 20 oszillierend in der Bewegungsrichtung 32 und in die Gegenrichtung.

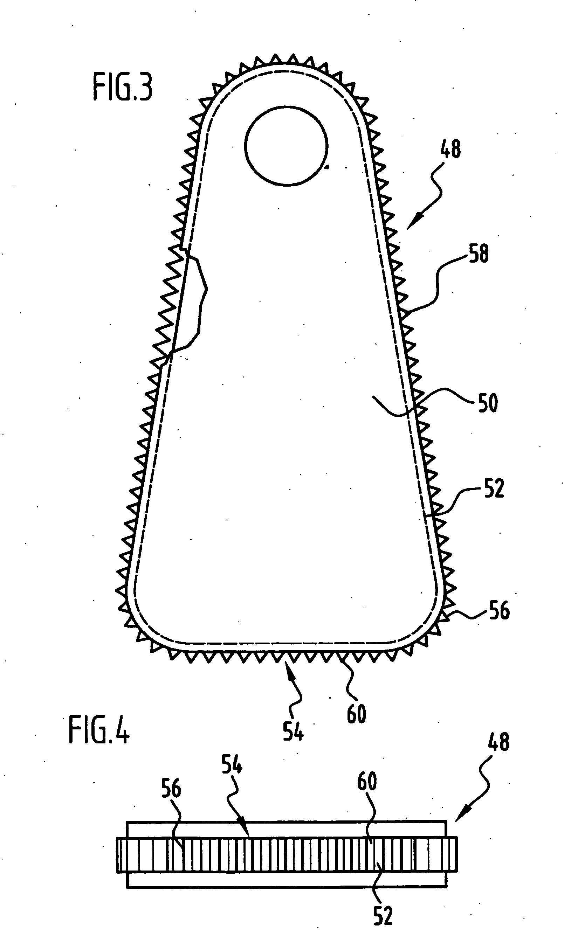

Bei einem zweiten Ausführungsbeispiel eines chirurgischen Sägeblattes, welches nicht unter die Ansprüche fällt und in den Figuren 3 und 4 gezeigt und dort als Ganzes mit 48 bezeichnet ist, ist wiederum ein Haltekörper 50 vorgesehen, welcher grundsätzlich gleich ausgebildet ist wie der oben beschriebene Haltekörper 12. Der Haltekörper 50 weist dabei eine umlaufende Nut 52 auf, wobei im Gegensatz zu dem Haltekörper 12 die Nut 52 auch im Bereich eines Sägeendes 54 gebildet ist.

In der Nut 52 ist als Sägeeinrichtung eine Sägekette 56 geführt. In Verbindung mit der Säge ist damit eine Kettensäge gebildet.

Die Sägekette 56 ist eine Endloseinrichtung. Sie umfasst eine Mehrzahl von Kettengliedern 58, wobei benachbarte Kettenglieder schwenkbar um eine Achse senkrecht zu der Oberfläche des Haltekörpers 50 verbunden sind. Damit kann sich die Sägekette 56 flexibel in der Nut 52 anlegen.

Die Sägekette 56 wiederum weist eine Vielzahl von Sägezähnen 60 auf, wobei pro Kettenglied 58 ein oder mehrere Sägezähne 60 gebildet sind.

Durch den Antrieb der Säge wird die Sägekette 56 relativ zu dem Haltekörper 50 bewegt. Als Kraftübertragungselement ist beispielsweise ein Zahnrad vorgesehen, welches von dem Sägenantrieb angetrieben ist und in die Sägekette 56 eingreift, um diese zu bewegen. Dadurch lässt sich ein Sägeschnitt einbringen. Bei der Bewegung kann es sich um eine um den Haltekörper 50 umlaufende Bewegung handeln oder um eine am Sägeende 54 oszillierende Bewegung.

Auch hier ist die Masse der bewegten Teile beim Einbringen eines Sägeschnitts minimiert, da nur die Sägekette 56 bewegt wird.

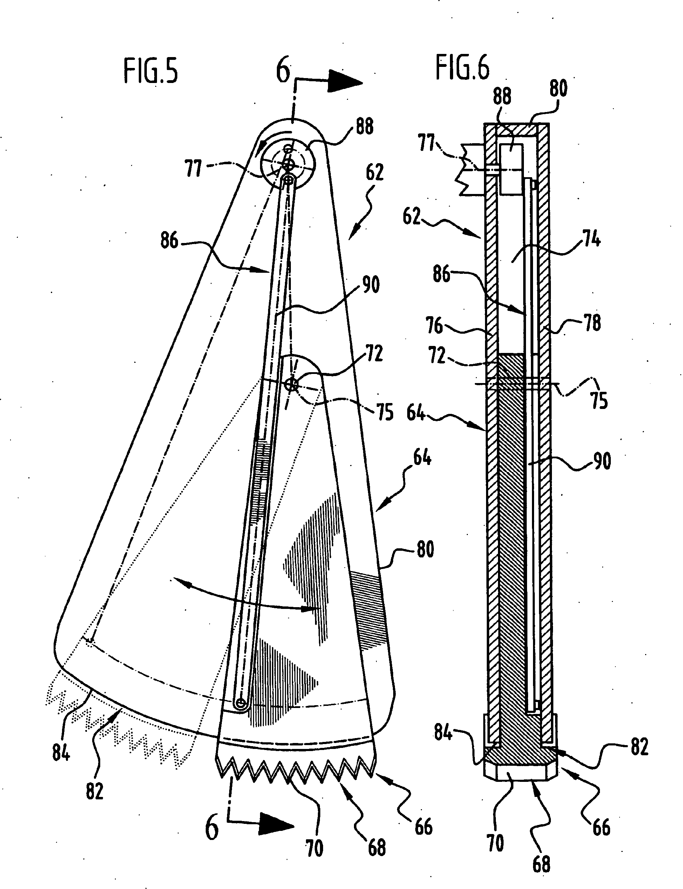

Bei einem dritten Ausführungsbeispiel eines chirurgischen Sägeblattes, welches in den Figuren 5 und 6 gezeigt ist und dort als Ganzes mit 62 bezeichnet ist, ist ein Haltekörper 64 vorgesehen, an welchem ein Sägekopf 66 beweglich und insbesondere schwenkbar angeordnet ist. Der Sägekopf 66 umfasst eine Zahnreihe 68 mit Sägezähnen 70. Diese Sägezähne 70 sind beispielsweise in einer Linie angeordnet.

Der Sägekopf 66 ist über ein Schwenklager 72 schwenkbar an dem Haltekörper 64 gelagert. Das Schwenklager 72 ist dabei in einem Innenraum 74 des Haltekörpers 64 angeordnet, welcher zwischen äußeren Deckelplatten 76, 78 des Haltekörpers 64 gebildet ist. Zwischen den Deckelplatten 76, 78 ist eine umlaufenden Seitenwand 80 angeordnet, welche den Innenraum 74 zur Seite hin abdeckt. Damit ist das Schwenklager 72 verdeckt in dem Innenraum 74 des Haltekörpers 64 angeordnet und gegenüber dem Außenraum geschützt. Eine Schwenkachse 75 des Schwenklagers 72 ist beabstandet zu einer Antriebsachse 77 angeordnet.

An seinem Sägeende 82 ist der Haltekörper 64 offen und mit einem Schlitz 84 versehen, in welchem der Sägekopf 66 schwenkbar ist. Der Haltekörper 64 umgreift dadurch den Sägekopf 66. Bei einem Oszillationssägevorgang ist der Haltekörper 64 nicht bewegt, so dass er führbar ist oder auch einspannbar ist.

Der Sägekopf 66 weist in Draufsicht eine dreiecksförmige Gestalt auf, wobei an einer Basisseite die Zahnreihe 68 sitzt und in dem gegenüberliegenden Eckbereich der Sägekopfteil (Welle oder Wellenaufnahme) des Schwenklagers 72 sitzt. Zur Kraftübertragung auf den Sägekopf 66 und Antrieb einer Oszillationsbewegung des Sägekopfes 66 ist als Umsetzungstrieb (Zwischengetriebe) ein Pleueltrieb 86 vorgesehen, welcher an den Antrieb der Säge (mit Antriebsachse 77) koppelbar ist.

Zahnspitzen der Zahnreihe 68 liegen auf einer Kreislinie, wobei der Mittelpunkt des entsprechenden Kreises mit der Schwenkachse 75 zusammenfällt.

Dazu ist in dem Innenraum 74 ein Antriebsrad 88 drehbar gelagert. Eine Drehachse dieses Antriebsrads 88 ist dabei parallel zu der Schwenkachse des Schwenklagers 72 ausgerichtet. Dieses Antriebsrad 88 ist über den Antrieb der Säge in Rotation versetzbar.

An das Antriebsrad 88 ist exzentrisch eine Pleuelstange 90 gekoppelt, welche wiederum an den Sägekopf 66 gekoppelt ist. Durch eine Rotation des Antriebsrads 88 wird der Sägekopf 66 über die Pleuelstange 90 in eine Oszillationsbewegung versetzt.

Über die Oszillationsbewegung des Sägekopfes 66 mit der Zahnreihe 68 lässt sich ein Sägeschnitt einbringen. Der Sägekopf 66 wird als Sägeeinrichtung gegenüber dem Haltekörper 64 bewegt, wobei der Pleueltrieb 86 Kraftübertragungselemente bereitstellt, an die der Sägenantrieb koppelbar ist, um die Schwenkbewegung zu bewirken. Die bewegte Masse ist verringert, da nur eine Bewegung des Sägekopfes 66 und des Pleueltriebs 86 bewirkt wird, nicht jedoch des ganzen Haltekörpers 64.

Weiterhin kann durch die Trennung zwischen dem Haltekörper 64 und dem Sägekopf 66 eine getrennte Optimierung durchgeführt werden. Beispielsweise lassen sich auch längere Sägeblätter ausbilden, um tiefere Schnitte einbringen zu können, da die Antriebsachse 77 und die Schwenkachse 75 beabstandet sind. Auch bei längeren Sägeblättern ist der Sägeweg nur durch die Schwenkbewegung des Sägekopfs 66, welcher an dem Haltekörper 64 gehalten ist, bestimmt. Dieser Weg ist aber unabhängig von der Länge des Sägeblatts. Weiterhin ist die bewegte Masse im Wesentlichen nur durch den Sägekopf 66 (und die Pleuelstange 90) bestimmt und nicht durch den Haltekörper 64 selber bestimmt.

Beispielsweise ist es auch möglich, den Sägekopf 66 mit der Zahnreihe 70 seitlich anzuordnen; bei dem Ausführungsbeispiel gemäß Figur 5 oszilliert der Sägekopf 66 um eine Längsachse des Sägeblatts 62. Es ist aber auch möglich, dass bei einer seitlichen Anordnung der Sägekopf um eine Querachse zu der Längsachse des Sägeblatts oszilliert. Eine solche seitliche Anordnung kann bei bestimmten chirurgischen Eingriffen vorteilhaft sein.

Es ist günstigerweise vorgesehen, dass die Breite der Zähne 68 (das heißt deren Länge parallel zur Achsenrichtung der Schwenkachse 75) mindestens so groß ist wie die entsprechende Breite des Haltekörpers 62, das heißt dass die Zähne bezüglich Außenseiten des Haltekörpers 62 nicht gegenüber diesem zurückgesetzt sind.

Im Zusammenhang mit den Figuren 5 und 6 wurde ein Pleueltrieb als Umsetzungstrieb zum Antreiben der Oszillationsbewegung des Sägekopfs 66 beschrieben. Insbesondere wird ein Exzentertrieb vorgesehen. Der entsprechende Mechanismus des Pleueltriebs 66 ist vollständig in dem Innenraum 74 des Haltekörpers 64 angeordnet.

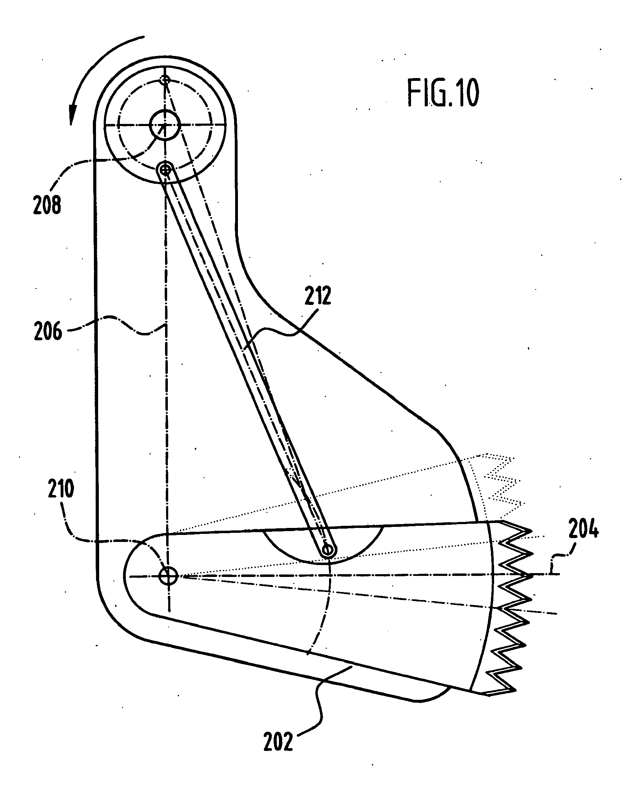

Bei einer alternativen Ausführungsform, welche in Figur 10 gezeigt ist, ist ein Sägekopf 202 seitlich angeordnet, das heißt eine Achse 204 (als "Nulllagenachse"), um welche der Sägekopf 202 oszilliert, fällt nicht mit einer Achse 206 zusammen, welche eine Antriebsachse 208 und eine Schwenkachse 210 in ihren entsprechenden Durchstoßpunkten verbindet. (Bei dem Ausführungsbeispiel gemäß den Figuren 5 und 6 oszilliert der Sägekopf 66 um die Verbindungslinie der Durchstoßpunkte der Drehachse 77 und der Schwenkachse 75.)

Der Umsetzungstrieb zum Antreiben der Oszillationsbewegung des Sägekopfs 202 ist grundsätzlich gleich ausgebildet wie der Pleueltrieb 66, welcher im Zusammenhang mit den Figuren 5 und 6 beschrieben wurde, wobei jedoch eine entsprechende Pleuelstange 212 so an dem Sägekopf 202 angelenkt ist, dass dieser eben um die Achse 204 oszillieren kann.

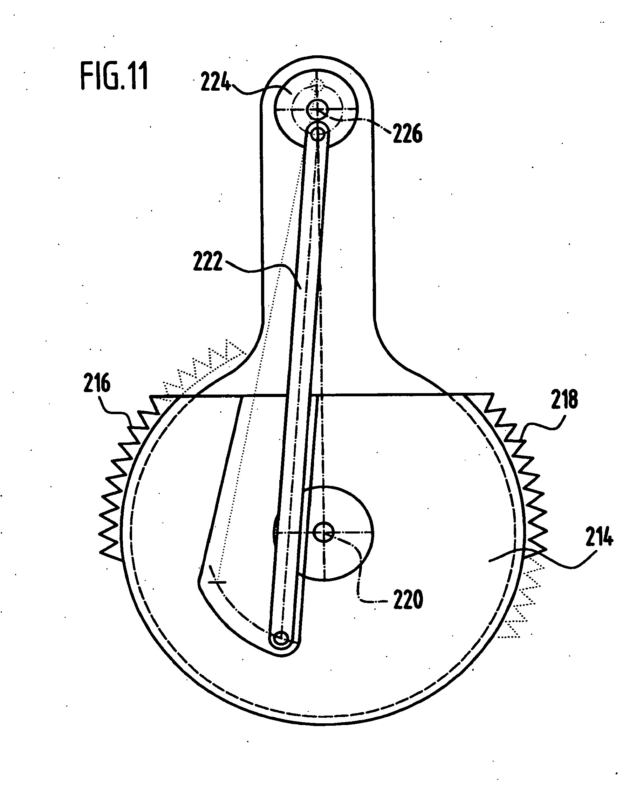

Bei einem weiteren Ausführungsbeispiel, welches schematisch in Figur 11 gezeigt ist, ist ein Sägekopf 214 vorgesehen, welcher beispielsweise kreisscheibenförmig ausgebildet ist. Der Sägekopf 214 umfasst eine erste Zahnreihe 216 und eine gegenüberliegende zweite Zahnreihe 218. Die beiden Zahnreihen 216 und 218 sitzen um 90° versetzt an dem Sägekopf 214.

Eine Schwenkachse 220 des Sägekopfs 214 geht durch einen Kreismittelpunkt des Sägekopfs 214. Eine Pleuelstange 222 ist exzentrisch an dem Sägekopf 214 angelenkt. Die Pleuelstange 222 wiederum wird über ein rotierbares Antriebselement 224 mit einer Rotationsachse 26 bewegt, wobei die Pleuelstange 222 exzentrisch an dem Antriebselement 224 angelenkt ist. Durch Rotation des Antriebselements 224, wobei diese Rotation durch einen Motor angetrieben ist, wird der Sägekopf 214 in eine Oszillationsbewegung versetzt, wobei über die Oszillationsbewegung sowohl die erste Zahnreihe 216 als auch die zweite Zahnreihe 218 oszilliert.

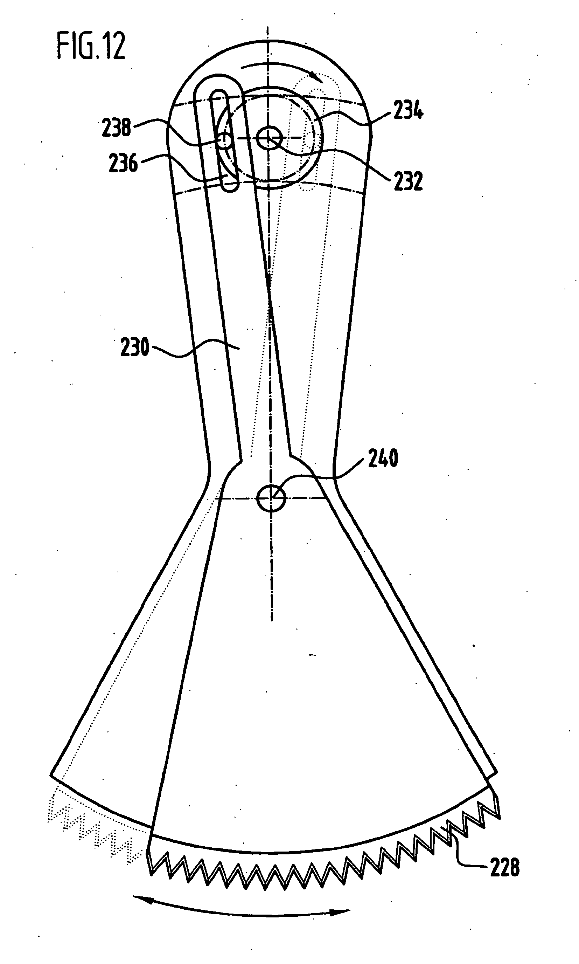

Bei einem weiteren Ausführungsbeispiel, welches in Figur 12 schematisch gezeigt ist, ist ein Sägekopf 228 vorgesehen, an welchem starr und beispielsweise einstückig eine Antriebsstange 230 angeordnet ist. Diese Antriebsstange 230 ist an einem um eine Rotationsachse 232 rotierbaren Antriebselement 234 exzentrisch angelenkt. Beispielsweise umfasst die Antriebsstange 230 dazu ein Langloch 236, in das ein Stiftelement 238 eintaucht, wobei das Stiftelement 238 exzentrisch an dem Antriebselement 234 sitzt.

Der Sägekopf 228 selber ist über ein Schwenklager 240 an einem entsprechenden Haltekörper schwenkbar gelagert. Durch Rotation des Antriebselements 234 wird dann über den entsprechenden Umsetzungstrieb der Sägekopf 228 in eine Oszillationsbewegung versetzt.

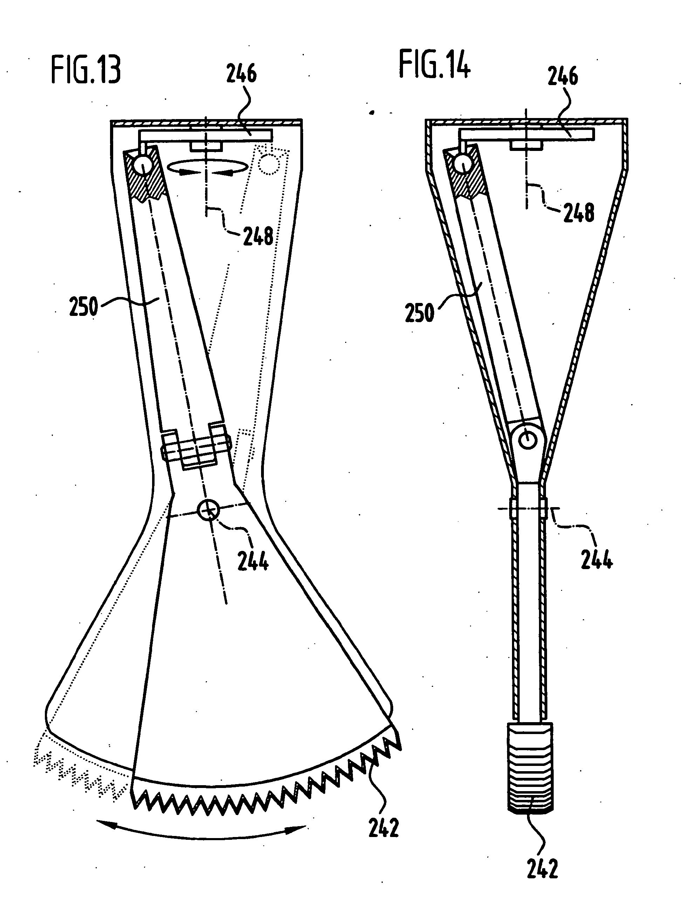

In den Figuren 13 und 14 ist ein weiteres Beispiel für einen Umsetzungsantrieb gezeigt. Ein Sägekopf 242 ist um eine Schwenkachse 244 schwenkbar gelagert. Ein Antriebselement 246 weist eine Rotationsachse 248 auf, welche quer und insbesondere senkrecht zu der Schwenkachse 244 liegt.

Ein Kopplungselement 250 ist an dem Sägekopf 242 angelenkt und exzentrisch an dem Antriebselement 246 angelenkt. Wenn das Antriebselement 246 um die Rotationsachse 248 rotiert, dann wird das Kopplungselement 250 mitrotiert. Die Anlenkung des Kopplungselement 250 an dem Sägekopf 242 ist derart, dass durch diese Bewegung des Kopplungselements 250 der Sägekopf 242 in eine Oszillationsbewegung um die Schwenkachse 242 angetrieben ist.

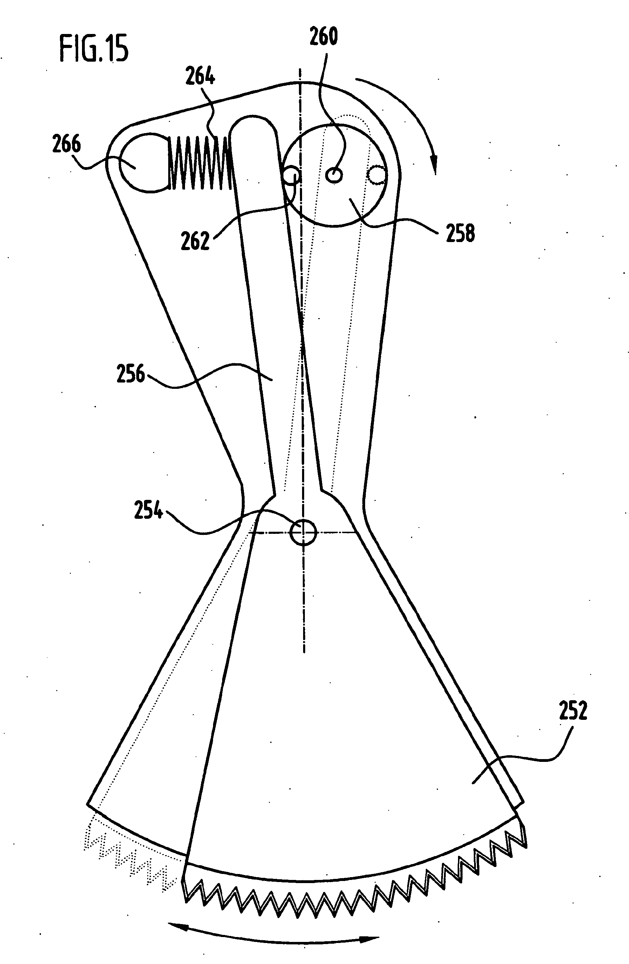

Bei einem weiteren Ausführungsbeispiel, welches in Figur 15 gezeigt ist, ist an einem Sägekopf 252, welcher um eine Schwenkachse 254 schwenkbar gelagert ist, starr und beispielsweise einstückig ein Kopplungselement 256 angeordnet.

Es ist ein Antriebselement 258 vorgesehen, welches um eine Rotationsachse 260 rotierbar ist. Die Rotationsachse 260 ist dabei im Wesentlichen parallel zur Schwenkachse 254.

An dem Antriebselement 258 sitzt exzentrisch ein Stiftelement 262, welches auf eine Oberfläche des Kopplungselements 256 wirkt.

Über eine Feder 264, welche sich an einem Flanschelement 266 abstützt, wird das Kopplungselement 256 gegen das Stiftelement 262 gedrückt. Dadurch wird dafür gesorgt, dass das Stiftelement 262 ständig auf das Kopplungselement 256 wirkt und dadurch wiederum die Rotationsbewegung des Antriebselements 258 auf das Kopplungselement 256 wirkt. Das Kopplungselement 256 wird dadurch in eine Oszillationsbewegung versetzt, die zu einer Oszillation des Sägekopfs 252 führt.

Das Flanschelement 266 ist unbeweglich in einem entsprechenden Haltekörper angeordnet.

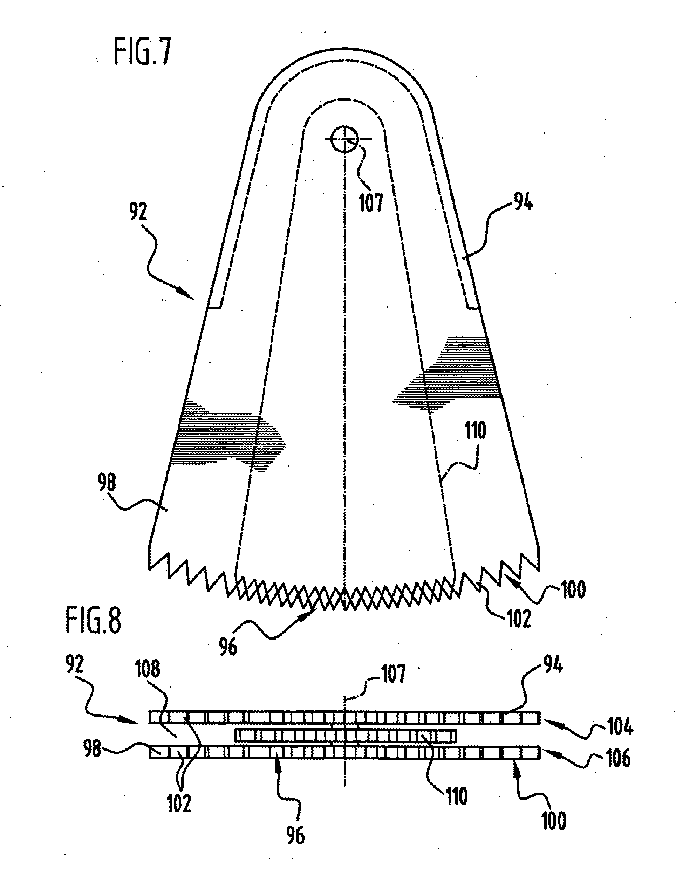

Bei einem vierten Ausführungsbeispiel eines erfindungsgemäßen chirurgischen Sägeblattes, welches in den Figuren 7 und 8 gezeigt und dort als Ganzes mit 92 bezeichnet ist, ist ein Haltekörper 94 vorgesehen, welcher an die Säge koppelbar ist. Dieser Haltekörper 94 weist ein Sägeende 96 auf, über welches sich ein Sägeschnitt einbringen lässt.

An dem Haltekörper 94 ist dabei an dem Sägeende 96 ein (erster) Sägekopf 98 angeordnet, welcher insbesondere einstückig an dem Haltekörper 94 ausgebildet ist. Dieser Sägekopf 98 umfasst eine Zahnreihe 100 mit Sägezähnen 102. Die Zahnreihe 100 weist eine obere Ebene 104 und eine untere Ebene 106 auf, wobei die Sägezähne 102 der oberen Ebene 104 und der unteren Eben 106 vorzugsweise bezüglich Zahnspitzen fluchtend aufeinander ausgerichtet sind.

Die Zahnspitzen liegen auf einem Kreis, dessen Kreismittelpunkt durch eine Antriebsachse 107 bestimmt ist.

Zwischen der oberen Ebene 104 und der unteren Ebene 106 ist eine schlitzförmige Ausnehmung 108 gebildet, in welcher ein zweiter Sägekopf 110 beweglich ist, wobei dieser zweite Sägekopf 110 insbesondere eine Oszillationsbewegung um die Achse 107 durchführen kann. Der Haltekörper 94 umgreift diesen Sägekopf 110. Der Sägekopf 98 kann eine Oszillationsbewegung durchführen, wenn der Haltekörper 94 eine Oszillationsbewegung durchführt. Der beweglich relativ zum Haltekörper 94 angeordnete zweite Sägekopf 110 kann an dem Haltekörper 94 eine Oszillationsbewegung durchführen, wenn er entsprechend angetrieben wird. Der zweite Sägekopf 110 kann dabei über einen Pleueltrieb oder andere Kraftübertragungselemente angetrieben werden, wie es oben im Zusammenhang mit dem dritten Ausführungsbeispiel 62 beschrieben wurde.

Insbesondere ist es vorgesehen, dass der erste Sägekopf 98 und der zweite Sägekopf 110 sich gegenläufig bewegen. Dadurch lässt sich die Sägefrequenz erhöhen und damit das Schnittergebnis verbessern.

Die Zahnspitzen des Sägekopfs 110 liegen ebenfalls auf einem Kreis, wobei dieser vorzugsweise den gleichen Radius und den gleichen Kreismittelpunkt wie der Kreis für die Zahnspitzen des Sägekopfs 98 aufweist.

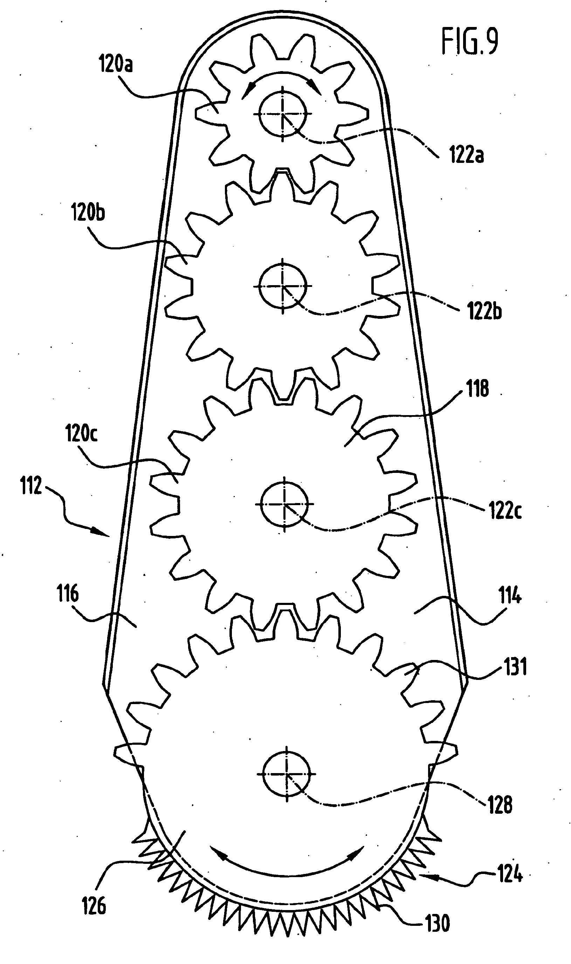

Bei einem fünften Ausführungsbeispiel, welches nicht unter die Ansprüche fällt und in Figur 9 schematisch gezeigt und dort als Ganzes mit 112 bezeichnet ist, ist ein Haltekörper 114 vorgesehen, welcher einen nach außen verdeckten Inneraum 116 aufweist, in welchem als Antriebselement mindestens ein Zahnrad 118 angeordnet ist. Bei dem in Figur 9 gezeigten Ausführungsbeispiel sind drei Zahnräder 120a, 120b und 120c als Antriebselemente vorgesehen. Dieser Zahnräder 120a, 120b und 120c sind dabei kaskadenartig angeordnet:
Die Zahnräder 120a, 120b, 120c sind in dem Innenraum 116 drehbar gelagert, wobei sie jeweils Drehachsen 122a, 122b, 122c aufweisen, die parallel zueinander ausgerichtet sind und deren Durchstoßpunkte auf einer Linien liegen. Diese Drehachsen 122a, 122b, 122c sind quer zu einer Oberfläche des Haltekörpers 114 und insbesondere senkrecht zu dieser Oberfläche ausgerichtet (parallel zu einer Normalenrichtung der Oberfläche).

Das Zahnrad 120a, welches am weitesten entfernt von einem Sägeende 124 des Haltekörpers 114 ist, weist den kleinsten Durchmesser auf. Das benachbarte Zahnrad 120b weist einen größeren Durchmesser auf und das noch näher am Sägeende 124 liegende Zahnrad 120c weist einen noch größeren Durchmesser auf.

Das Zahnrad 120a wird über den Antrieb der Säge in einer Rotationsbewegung oder Oszillationsbewegung angetrieben. Diese Bewegung wird übertragen auf das Zahnrad 120b, das heißt dieses wird ebenfalls in eine Rotationsbewegung oder Oszillationsbewegung versetzt. Das entsprechende Drehmoment wird wiederum auf das Zahnrad 120c übertragen. Das Zahnrad 120c treibt dann einen Sägekopf 126 an, welcher zumindest teilweise als Zahnrad ausgebildet ist. Dieser Sägekopf 126 ist über ein Drehlager drehbar an dem Haltekörper 114 gelagert, wobei eine Drehachse 128 parallel zu den Drehachsen 122a, 122b, 122c ausgerichtet ist und ein Durchstoßpunkt auf der gleichen Linie wie der Durchstoßpunkt dieser Drehachsen liegt. Der Sägekopf 126 stellt gewissermaßen ein Kreissägeblatt dar.

Er ist dazu mit Sägezähnen 130 versehen, die um den Umfang des Sägekopfes 126 mindestens in einem Teilbereich gleichmäßig verteilt angeordnet sind. Es können Zahnradzähne 131 zum Eingriff in das Zahnrad 120c vorgesehen sein.

Der Sägekopf 126 ist außer rotierend auch oszillierend antreibbar, indem entsprechend das Zahnrad 120a oszillierend angetrieben wird. Der Sägekopf 126 ist dann vorzugsweise so ausgebildet, dass nur die Zahnradzähne 131 (und nicht die Sägezähne 130) an das Zahnrad 120c angreifen.

Der Sägekopf 126 bildet eine Sägeeinrichtung, welche relativ zu dem Haltekörper 114 beweglich ist. Auch hier muss nicht der gesamte Haltekörper 114 bewegt werden. Die Zahnräder 120a, 120b, 120c bilden ein Zwischengetriebe als Umsetzungstrieb, über welches die Antriebskraft des Antriebs der Säge auf den Sägekopf 126 übertragbar ist. Ein entsprechendes Drehmoment lässt sich über einen Kopplungsbereich des Haltekörpers 114 einkoppeln, wobei dieser Kopplungsbereich in der Nähe eines dem Sägeende abgewandten Endes des Haltekörpers 114 angeordnet ist.

## Patentansprüche

1. Chirurgisches Sägeblatt mit einem Haltekörper (64; 94) und einer Sägeeinrichtung (66; 110), welche an dem Haltekörper (64; 94) gehalten ist und relativ zu dem Haltekörper (64; 94) beweglich ist, wobei die Sägeeinrichtung einen schwenkbaren, eine oszillierende Bewegung durchführbaren Sägekopf (66; 110; 202; 214; 228; 242; 252) umfasst,
**dadurch gekennzeichnet, dass** die Sägeeinrichtung (66) über den Haltekörper (64; 94) an einen Antrieb zur Bewegung der Sägeeinrichtung (66) gekoppelt ist, wobei der Haltekörper (64; 94) bei einem Oszillationssägevorgang nicht bewegt ist, dass der Haltekörper (64; 94) einen Kopplungsbereich umfasst, über den ein externer Antrieb wirksam an ein Kraftübertragungselement oder an Kraftübertragungselemente (86; 212; 222; 230; 250; 256) zur Bewegung der Sägeeinrichtung (66; 110) koppelbar ist, dass das oder die Kraftübertragungselemente (86; 212; 222; 230; 250; 256) zwischen gegenüberliegenden äußeren Oberflächen (16, 18) des Haltekörpers (80) angeordnet sind und dass der Haltekörper (64; 94) an einem Sägeende (82; 96) mit einem Schlitz (84; 108) versehen ist, in welchem der Sägekopf (66; 110; 202; 214; 228; 242; 252) schwenkbar ist.

2. Chirurgisches Sägeblatt nach Anspruch 1, **dadurch gekennzeichnet, dass** der Kopplungsbereich eine Öffnung an dem Haltekörper (64; 94) umfasst.

3. Chirurgisches Sägeblatt nach einem der vorangehenden Ansprüche, **dadurch gekennzeichnet, dass** der Haltekörper (64; 94) an dem Sägeende (82; 96) eine größere Breite aufweist als an einem gegenüberliegenden Ende.

4. Chirurgisches Sägeblatt nach einem der vorangehenden Ansprüche, **dadurch gekennzeichnet, dass** sich die Sägeeinrichtung (66; 110) an dem Sägeende (82; 96) des Haltekörpers (64; 94) quer zu einer Längsrichtung des Haltekörpers (64; 94) bewegt.

5. Chirurgisches Sägeblatt nach einem der vorangehenden Ansprüche,
**dadurch gekennzeichnet, dass** der Sägekopf (66; 110; 202; 214; 228; 242; 252) schwenkbar in dem Haltekörper (64; 94) gehalten ist.

6. Chirurgisches Sägeblatt nach einem der vorangehenden Ansprüche,
**dadurch gekennzeichnet, dass** der Sägekopf (66; 110; 202; 214; 228; 242; 252) von dem Haltekörper (64; 94) umgriffen ist.

7. Chirurgisches Sägeblatt nach einem der vorangehenden Ansprüche,
**dadurch gekennzeichnet, dass** der Sägekopf (66; 110; 202; 214; 228; 242; 252) eine Zahnreihe (68) aufweist.

8. Chirurgisches Sägeblatt nach Anspruch 7, **dadurch gekennzeichnet, dass** die Zähne (70) der Zahnreihe (68) mindestens so hoch sind wie der Haltekörper (64).

9. Chirurgisches Sägeblatt nach Anspruch 7 oder 8, **dadurch gekennzeichnet, dass** Spitzen der Zähne (70; 102) der Zahnreihe (68; 98) auf einer Kreislinie liegen.

10. Chirurgisches Sägeblatt nach Anspruch 9, **dadurch gekennzeichnet, dass** der Mittelpunkt der Kreislinie auf einer Schwenkachse (75) für den Sägekopf (66) liegt.

11. Chirurgisches Sägeblatt nach einem der vorangehenden Ansprüche, **dadurch gekennzeichnet, dass** eine Antriebsachse (77) für den Schwenkantrieb des Sägekopfs (66) und eine Schwenkachse (75) beabstandet sind.

12. Chirurgisches Sägeblatt nach einem der vorangehenden Ansprüche, **dadurch gekennzeichnet, dass** ein Umsetzungstrieb (86) zur Umsetzung einer Antriebsrotationsbewegung in eine Sägekopf-Schwenkbewegung vorgesehen ist.

13. Chirurgisches Sägeblatt nach Anspruch 12, **dadurch gekennzeichnet, dass** der Umsetzungstrieb als Exzentertrieb (88) ausgebildet ist.

14. Chirurgisches Sägeblatt nach Anspruch 12 oder 13, **dadurch gekennzeichnet, dass** der Mechanismus des Umsetzungstriebs im Haltekörper (64) angeordnet ist.

15. Chirurgisches Sägeblatt nach einem der vorangehenden Ansprüche, **dadurch gekennzeichnet, dass** der Sägekopf (66) an einen Pleueltrieb (86) gekoppelt ist, welcher an dem Haltekörper (64) angeordnet ist.

16. Chirurgisches Sägeblatt nach einem der vorangehenden Ansprüche, **dadurch gekennzeichnet, dass** der Sägekopf (66) so an dem Haltekörper (64) angeordnet ist, dass eine Schwenkbewegung relativ zu einem vorderen Ende des Haltekörpers (64) durchführbar ist.

17. Chirurgisches Sägeblatt nach einem der vorangehenden Ansprüche, **dadurch gekennzeichnet, dass** der Sägekopf (202) so an dem Haltekörper angeordnet ist, dass eine Schwenkbewegung relativ zu einem seitlichen Ende des Haltekörpers durchführbar ist.

18. Chirurgisches Sägeblatt nach einem der vorangehenden Ansprüche, **dadurch gekennzeichnet, dass** ein erster Sägekopf (98) und ein zweiter Sägekopf (110) vorgesehen sind, wobei die beiden Sägeköpfe (98, 110) relativ zueinander beweglich sind.

19. Chirurgisches Sägeblatt nach Anspruch 18, **dadurch gekennzeichnet, dass** der erste Sägekopf (98) fest mit dem Haltekörper (94) verbunden ist.

20. Chirurgisches Sägeblatt nach Anspruch 18 oder 19, **dadurch gekennzeichnet, dass** der erste Sägekopf (98) den zweiten Sägekopf (110) zumindest teilweise umgibt.

21. Chirurgisches Sägeblatt nach Anspruch 20, **dadurch gekennzeichnet, dass** der erste Sägekopf (98) eine Ausnehmung (108) aufweist, in welcher der zweite Sägekopf (110) angeordnet ist.

22. Chirurgisches Sägeblatt nach einem der Ansprüche 18 bis 21, **dadurch gekennzeichnet, dass** der zweite Sägekopf (110) relativ zum Haltekörper (94) beweglich ist.

23. Chirurgisches Sägeblatt nach einem der Ansprüche 18 bis 22, **dadurch gekennzeichnet, dass** erster Sägekopf (98) und zweiter Sägekopf (110) gegenläufig beweglich sind.

## Claims

1. A surgical saw blade having a holding body (64; 94) and a sawing device (66; 110) which is held on the holding body (64; 94) and is movable relative to the holding body (64; 94), wherein the sawing device comprises a pivotable saw head (66; 110; 202; 214; 228; 242; 252) able to execute an oscillating motion, **characterised in that** the sawing device (66) is coupled, via the holding body (64; 94), to a drive for movement of the sawing device (66), wherein the holding body (64; 94) is not moved during an oscillation sawing procedure, **in that** the holding body (64; 94) comprises a coupling region via which an external drive can be operatively coupled to a force transmission element or to force transmission elements (86; 212; 222; 230; 250; 256) for movement of the sawing device (66; 110), **in that** the force transmission element(s) (86; 212; 222; 230; 250; 256) are arranged between facing, outer surfaces (16, 18) of the holding body (80), and **in that** the holding body (64; 94) is provided at one saw end (82; 96) with a slot (84; 108) in which the saw head (66; 110; 202; 214; 228; 242; 252) is pivotable.

2. A surgical saw blade according to claim 1, **characterised in that** the coupling region comprises an opening at the holding body (64; 94).

3. A surgical saw blade according to any one of the preceding claims, **characterised in that** the holding body (64; 94) is greater in width at the saw end (82; 96) than at an opposite end.

4. A surgical saw blade according to any one of the preceding claims, **characterised in that** at the saw end (82; 96) of the holding body (64; 94), the sawing device (66; 110) moves transversely to a longitudinal direction of the holding body (64; 94).

5. A surgical saw blade according to any one of the preceding claims, **characterised in that** the saw head (66; 110; 202; 214; 228; 242; 252) is held in the holding body (64; 94) in a pivotable manner.

6. A surgical saw blade according to any one of the preceding claims, **characterised in that** the saw head (66; 110; 202; 214; 228; 242; 252) is embraced by the holding body (64; 94).

7. A surgical saw blade according to any one of the preceding claims, **characterised in that** the saw head (66; 110; 202; 214; 228; 242; 252) has a row of teeth (68).

8. A surgical saw blade according to claim 7, **characterised in that** the teeth (70) of the row of teeth (68) are at least as high as the holding body (64).

9. A surgical saw blade according to claim 7 or 8, **characterised in that** apexes of the teeth (70; 102) of the row of teeth (68; 98) lie on a circular curve.

10. A surgical saw blade according to claim 9, **characterised in that** the midpoint of the circular curve lies on a pivot axis (75) for the saw head (66).

11. A surgical saw blade according to any one of the preceding claims, **characterised in that** a drive axis (77) to drive the saw head (66) so as to pivot and a pivot axis (75) are at a distance from one another.

12. A surgical saw blade according to any one of the preceding claims, **characterised in that** a conversion drive (86) for converting a drive rotational motion into a saw-head pivoting motion is provided.

13. A surgical saw blade according to claim 12, **characterised in that** the conversion drive is in the form of an eccentric drive (88).

14. A surgical saw blade according to claim 12 or 13, **characterised in that** the mechanism of the conversion drive is arranged in the holding body (64).

15. A surgical saw blade according to any one of the preceding claims, **characterised in that** the saw head (66) is coupled to a connecting-rod drive (86) arranged on the holding body (64).

16. A surgical saw blade according to any one of the preceding claims, **characterised in that** the saw head (66) is arranged on the holding body (64) in such a manner that a pivoting motion relative to a front end of the holding body (64) can be executed.

17. A surgical saw blade according to any one of the preceding claims, **characterised in that** the saw head (202) is arranged on the holding body in such a manner that a pivoting motion relative to a lateral end of the holding body can be executed.

18. A surgical saw blade according to any one of the preceding claims, **characterised in that** a first saw head (98) and a second saw head (110) are provided, wherein the two saw heads (98, 110) are movable relative to one another.

19. A surgical saw blade according to claim 18, **characterised in that** the first saw head (98) is securely connected to the holding body (94).

20. A surgical saw blade according to claim 18 or 19, **characterised in that** the first saw head (98) at least partially surrounds the second saw head (110).

21. A surgical saw blade according to claim 20, **characterised in that** the first saw head (98) has an opening (108) in which the second saw head (110) is arranged.

22. A surgical saw blade according to any one of claims 18 to 21, **characterised in that** the second saw head (110) is movable relative to the holding body (94).

23. A surgical saw blade according to any one of claims 18 to 22, **characterised in that** the first saw head (98) and the second saw head (110) are movable in opposite directions.

## Revendications

1. Lame de scie chirurgicale comprenant un corps de support (64; 94) et un dispositif de sciage (66; 110) qui est supporté ou maintenu par le corps de support (64; 94) et est mobile par rapport au corps de support (64; 94), le dispositif de sciage englobant une tête de sciage (66; 110; 202; 214; 228; 242; 252) pouvant pivoter et susceptible d'effectuer un mouvement oscillant, **caractérisée en ce que** le dispositif de sciage (66) est couplé, par l'intermédiaire du corps de support (64; 94), à un système d'entraînement pour assurer le mouvement du dispositif de sciage (66), le corps de support (64; 94) n'étant pas déplacé lors d'une opération de sciage oscillant,
**en ce que** le corps de support (64; 94) comprend une zone de couplage au travers de laquelle un système d'entraînement externe peut être couplé de manière active à un élément de transmission de force ou à des éléments de transmission de force (86; 212; 222; 230; 250; 256) pour assurer le mouvement du dispositif de sciage (66; 110),
**en ce que** l'élément ou les éléments de transmission de force (86; 212; 222; 230; 250; 256) sont agencés entre des surfaces extérieures opposées (16, 18) du corps de support (80), et
**en ce que** le corps de support (64; 94) est, à une extrémité de sciage (82; 96), pourvu d'une fente (84; 108) dans laquelle peut pivoter la tête de sciage (66; 110; 202; 214; 228; 242; 252).

2. Lame de scie chirurgicale selon la revendication 1, **caractérisée en ce que** la zone de couplage comprend une ouverture dans le corps de support (64; 94).

3. Lame de scie chirurgicale selon l'une des revendications précédentes, **caractérisée en ce que** le corps de support (12; 94) présente, au niveau de l'extrémité de sciage (82; 96), une largeur plus grande qu'au niveau d'une extrémité opposée.

4. Lame de scie chirurgicale selon l'une des revendications précédentes, **caractérisée en ce que** le dispositif de sciage (66; 110) se déplace, au niveau de l'extrémité de sciage (82; 96) du corps de support (64; 94), transversalement à une direction longitudinale du corps de support (64; 94).

5. Lame de scie chirurgicale selon l'une des revendications précédentes, **caractérisée en ce que** la tête de sciage (66; 110; 202; 214; 228; 242; 252) est supportée ou maintenue de manière pivotante dans le corps de support (64; 94).

6. Lame de scie chirurgicale selon l'une des revendications précédentes, **caractérisée en ce que** la tête de sciage (66; 110; 202; 214; 228; 242; 252) est entourée par le corps de support (64; 94).

7. Lame de scie chirurgicale selon l'une des revendications précédentes, **caractérisée en ce que** la tête de sciage (66; 110; 202; 214; 228; 242; 252) présente une rangée de dents (58).

8. Lame de scie chirurgicale selon la revendication 7, **caractérisée en ce que** les dents (70) de la rangée de dents (68) ont une hauteur, à savoir une épaisseur au moins égale à celle du corps de support (64).

9. Lame de scie chirurgicale selon la revendication 7 ou la revendication 8, **caractérisée en ce que** les sommets ou pointes des dents (70; 102) de la rangée de dents (68; 98) se situent sur une ligne circulaire.

10. Lame de scie chirurgicale selon la revendication 9, **caractérisée en ce que** le centre de la ligne circulaire se situe sur un axe de pivotement (75) pour la tête de sciage (66).

11. Lame de scie chirurgicale selon l'une des revendications précédentes, **caractérisée en ce qu'**un axe d'entraînement (77) pour l'entraînement pivotant de la tête de sciage (66) et l'axe de pivotement (75) sont distants l'un de l'autre.

12. Lame de scie chirurgicale selon l'une des revendications précédentes, **caractérisée en ce qu'**il est prévu une transmission de conversion (86) pour convertir un mouvement de rotation d'entraînement en un mouvement de pivotement de la tête de sciage.

13. Lame de scie chirurgicale selon la revendication 12, **caractérisée en ce que** la transmission de conversion est réalisée sous forme de transmission à excentrique (88).

14. Lame de scie chirurgicale selon la revendication 12 ou la revendication 13, **caractérisée en ce que** le mécanisme de la transmission de conversion est agencé dans le corps de support (64).

15. Lame de scie chirurgicale selon l'une des revendications précédentes, **caractérisée en ce que** la tête de sciage (66) est couplée à une transmission à bielle (86), qui est agencée sur le corps de support (64).

16. Lame de scie chirurgicale selon l'une des revendications précédentes, **caractérisée en ce que** la tête de sciage (66) est agencée sur le corps de support (64) de manière à permettre un mouvement de pivotement par rapport à une extrémité avant du corps de support (64).

17. Lame de scie chirurgicale selon l'une des revendications précédentes, **caractérisée en ce que** la tête de sciage (202) est agencée sur le corps de support de manière à permettre un mouvement de pivotement par rapport à une extrémité latérale du corps de support.

18. Lame de scie chirurgicale selon l'une des revendications précédentes, **caractérisée en ce que** sont prévues une première tête de sciage (98) et une deuxième tête de sciage (110), les deux têtes de sciage (98, 110) étant mobiles l'une par rapport à l'autre.

19. Lame de scie chirurgicale selon la revendication 18, **caractérisée en ce que** la première tête de sciage (98) est repliée de manière fixe au corps de support (94).

20. Lame de scie chirurgicale selon la revendication 18 ou la revendication 19, **caractérisée en ce que** la première tête de sciage (98) entoure au moins partiellement la deuxième tête de sciage (110).

21. Lame de scie chirurgicale selon la revendication 20, **caractérisée en ce que** la première tête de sciage (98) présente un évidement (108) dans lequel est agencée la deuxième tête de sciage (110).

22. Lame de scie chirurgicale selon l'une des revendications 18 à 21, **caractérisée en ce que** la deuxième tête de sciage (110) est mobile par rapport au corps de support (94).

23. Lame de scie chirurgicale selon l'une des revendications 18 à 22, **caractérisée en ce que** la première tête de sciage (98) et la deuxième tête de sciage (110) sont mobiles de manière mutuellement opposée.
